Europäisches Patentamt

European Patent Office

Office européen des brevets

(11) Veröffentlichungsnummer: **0 467 068 A1**

## (12) EUROPÄISCHE PATENTANMELDUNG

(21) Anmeldenummer: **91109635.2**

(22) Anmeldetag: **12.06.91**

(51) Int. Cl.5: **A61M 16/18**

(30) Priorität: **14.07.90 DE 4022492**

(43) Veröffentlichungstag der Anmeldung:
**22.01.92 Patentblatt 92/04**

(84) Benannte Vertragsstaaten:
**DE FR GB IT**

(71) Anmelder: **Drägerwerk Aktiengesellschaft**
**Moislinger Allee 53-55**
**W-2400 Lübeck 1(DE)**

(72) Erfinder: **Wallroth, Carl-Friedrich, Dr.**
**Stresemannstrasse 1**
**W-2400 Lübeck 1(DE)**
Erfinder: **Falb, Wolfgang**
**Beidendorfer Weg 4**
**W-2401 Krummesse(DE)**
Erfinder: **Gippert, Karl-Ludwig, Dr.**
**Erlenkamp 15**
**W-2400 Lübeck 1(DE)**
Erfinder: **Hecker, Eric, Dr.**
**Hirschbergstrasse 2**
**W-2406 Stockelsdorf(DE)**

(54) **Füllvorrichtung für einen Narkosemittelverdunster mit temperaturempfindlichem Schaltglied.**

(57) Eine Füllvorrichtung zum Befüllen und Entleeren eines Narkosemittelverdunsters mit einer Narkoseflüssigkeit (2) aus einem Vorratsbehälter (1) über einen Füllkanal (3) und einen Belüftungskanal (4), mit einem Anschlußstück (6) zum Narkosemittelverdunster und mit einer Absperrvorrichtung (11), die im Leitungszug von Füllkanal (3) und Belüftungskanal (4) angeordnet ist, soll derart verbessert werden, daß eine gesicherte Handhabung bei niedrig siedenden Narkoseflüssigkeiten ermöglicht wird. Hierzu ist vorgesehen, daß die Absperrvorrichtung (11) ein temperaturempfindliches Schaltglied (12) besitzt, durch welches zumindest der Füllkanal (3) unterhalb einer vorgewählten Grenztemperatur in Öffnungsstellung und oberhalb der vorgewählten Grenztemperatur, jedoch unterhalb des Siedepunktes der Narkoseflüssigkeit (2), in Schließstellung bringbar ist.

FIG. 1

Die Erfindung betrifft eine Füllvorrichtung zum Befüllen und Entleeren eines Narkosemittelverdunsters mit einer Narkoseflüssigkeit aus einem Vorratsbehälter über einen Füllkanal und einem Belüftungskanal, mit einem Anschlußstück zum Narkosemittelverdunster und mit einer Absperrvorrichtung, die im Leitungszug von Füllkanal und Belüftungskanal angeordnet ist.

Eine Füllvorrichtung zum Befüllen und Entleeren eines Narkosemittelverdunsters mit Narkoseflüssigkeit über einem angeschlossenen Vorratsbehälter ist aus der DE-PS 37 20 326 bekanntgeworden. Sie besteht aus einem bewegbaren, rohrförmigen Leitungsstück, welches an einem Ende an den Vorratsbehälter angeschlossen ist und am anderen Ende ein Anschlußstück zum Eingriff in den Narkosemittelverdunster besitzt. Das rohrförmige Leitungsstück weist einen vom Füllkanal getrennten Belüftungskanal auf, wobei beide Kanäle als koaxiale Leitungen von dem Vorratsbehälter zum Anschlußstück geführt sind. Zum Befüllen wird das Anschlußstück des rohrförmigen Leitungsstücks zum Eingriff in den Narkosemittelverdunster gebracht und der Vorratsbehälter angehoben. Nach Betätigung eines Umschaltventils am Narkosemittelverdunster fließt die Narkoseflüssigkeit durch den hydrostatischen Druck über den Füllkanal in den Tank des Narkosemittelverdunsters, während gleichzeitig das aus dem Tank des Narkosemittelverdunsters verdrängte Gasvolumen über den Belüftungskanal in den Vorratsbehälter gelangt. Füllkanal und Belüftungskanal besitzen die Funktion von kommunizierenden Röhren zwischen Vorratsbehälter und dem Tank des Narkosemittelverdunsters. Nach Beendigung des Befüllvorgangs wird der Vorratsbehälter abgesenkt und das Umschaltventil am Narkosemittelverdunster wieder geschlossen. Zum Anheben und Absenken des Vorratsbehälters ist innerhalb des Leitungszuges des röhrenförmigen Leitungsstückes ein Drehgelenk vorgesehen, um das der Vorratsbehälter schwenkbar ist. In das Drehgelenk ist eine Absperrvorrichtung integriert, welche den Füllkanal und den Belüftungskanal verschließt, wenn der Vorratsbehälter nach dem Befüllen abgesenkt ist.

Bei der bekannten Vorrichtung ist es von Nachteil, daß die Absperrvorrichtung durch das Anheben und Absenken des Vorratsbehälters betätigt wird, unabhängig davon, welcher Dampfdruck sich durch die Narkoseflüssigkeit innerhalb der Vorratsbehälter gebildet hat. Befindet sich nämlich im Vorratsbehälter eine bei niedrigen Temperaturen siedende Narkoseflüssigkeit, kann ein Befüllen des Narkosemittelverdunsters auch schon bei üblichen Raumtemperaturen durch Blasenbildung unmöglich sein. Wird außerdem die Absperrvorrichtung versehentlich geöffnet, z.B. durch Drehen des röhrenförmigen Leitungsstückes, entweicht der in dem Vorratsbehälter befindliche dampfförmige Teil der Narkoseflüssigkeit in die Umgebung, und die Narkoseflüssigkeit beginnt durch den Druckabfall zu sieden. Nach kurzer Zeit kann so der gesamte Vorrat an Narkoseflüssigkeit verdampft sein.

Aus der DE-AS 1657174 ist eine weitere Füllvorrichtung zum Anschließen eines Vorratsbehälters an einen Narkosemittelverdunster bekanntgeworden. An der Verbindungsstelle zwischen Narkosemittelverdunster und Vorratsbehälter ist ein Kupplungssystem mit Ventilen vorgesehen, welche beim Kuppeln durch Kupplungsstifte geöffnet werden. Beim Befüllen fließt durch Schwerkrafteinwirkung die Narkoseflüssigkeit über einen Füllkanal in den Tank des Narkosemittelverdunsters, und über einen Belüftungskanal wird der Bodenraum des umgestülpten Vorratsbehälters belüftet.

Nachteilig bei der bekannten Vorrichtung ist, daß der Füllvorgang bei niedrig siedenden Narkoseflüssigkeiten beeinträchtigt ist, da das Einschalten des Füllvorgangs nur von der richtigen Überdeckung der entsprechenden Kupplungsstifte, nicht aber von der Temperatur der Narkoseflüssigkeit beeinflußt wird.

Der Erfindung liegt somit die Aufgabe zugrunde, eine Füllvorrichtung derart zu verbessern, daß eine gesicherte Handhabung bei niedrig siedenden Narkoseflüssigkeiten ermöglicht wird.

Die Lösung der Aufgabe erfolgt dadurch, daß die Absperrvorrichtung ein temperaturempfindliches Schaltglied besitzt, durch welches zumindestens der Füllkanal unterhalb einer vorgewählten Grenztemperatur in Öffnungsstellung und oberhalb der vorgewählten Grenztemperatur, jedoch unterhalb des Siedepunktes der Narkoseflüssigkeit, in Schließstellung bringbar ist.

Der Vorteil der Erfindung besteht im wesentlichen darin, daß die Absperrvorrichtung mit einem temperaturempfindlichen Schaltglied versehen ist, durch welches der Füllkanal erst unterhalb einer vorgewählten Grenztemperatur geöffnet wird und die Narkoseflüssigkeit erst dann bei umgestülptem Vorratsbehälter unter der Wirkung der Schwerkraft in den Tank des Narkosemittelverdunsters fließen kann. Oberhalb der Grenztemperatur wird die Absperrvorrichtung durch das Schaltglied in Schließstellung gebracht und der Narkoseflüssigkeitsstrom ist unterbrochen. Vor dem Befüllen müssen die Füllvorrichtung und der Vorratsbehälter auf einen Wert unterhalb der Grenztemperatur abgekühlt sein. Dieses kann so geschehen, daß beide Komponenten in einem Kühlschrank aufbewahrt und erst kurz vor dem Befüllen aus diesem entnommen werden. Zur Überwachung der Grenztemperatur kann die Vorratsflasche mit einem Temperaturindikator versehen sein, der anzeigt, daß die Grenztemperatur unterschritten ist. Die Absperrvorrichtung im Füllkanal kann als Hubventil mit einem axial beweglichen Verschlußteil auf einem Ventilsitz ausgeführt sein, wobei das Verschlußteil durch das Schaltglied betätigt ist. Alternative Absperrvorrichtungen sind Schieber, bei denen das Verschlußteil beim Öffnen und Schließen am Sitz entlanggleitet, Hähne mit einer Drehkörperfläche oder Membranabsperrventile.

In einer zweckmäßigen Ausgestaltung der Erfindung ist die Absperrvorrichtung als ein Füllventil im Füllkanal und ein Belüftungsventil im Belüftungskanal ausgeführt, welche gemeinsam durch das temperaturempfindliche Schaltglied betätigt werden. Durch das Vorhandensein einer Absperrvorrichtung sowohl im Füllkanal als auch im Belüftungskanal ist es möglich, den Vorratsbehälter vollständig abzusperren, wenn die Grenztemperatur überschritten ist. Der Vorratsbehälter kann so auch ohne separaten Behälterverschluß bei Temperaturen oberhalb der Grenztemperatur gelagert werden, ohne daß dampfförmige Narkosemittelflüssigkeit in die Umgebung entweicht.

Es ist vorteilhaft, die Absperrvorrichtung innerhalb des Vorratsbehälters anzuordnen, da das temperaturempfindliche Schaltglied dort direkt mit der Narkoseflüssigkeit in thermischem Kontakt steht. Ist der Vorratsbehälter zusätzlich in einem Isolierkörper angeordnet, ist so ein direkter Kontakt mit der Flüssig- und auch der Gasphase der Narkosemittelflüssigkeit ohne Beeinflussung von außen möglich. Mit der Absperrvorrichtung innerhalb des Vorratsbehälters kann so ein möglicher Austritt von Gas oder Flüssigkeit direkt am Vorratsbehälter unterbrochen werden.

In einer weiteren Ausführungsform ist die Absperrvorrichtung innerhalb oder in der Nähe des Anschlußstückes angeordnet. Diese Konfiguration besitzt den Vorteil, daß die Absperrvorrichtung erst dann in Öffnungsstellung ist, wenn sich der Bereich um das Anschlußstück auf einem Temperaturniveau unterhalb der vorgewählten Grenztemperatur, d.h. der Siedetemperatur der Narkoseflüssigkeit befindet. Durch diese Anordnung wird erreicht, daß sich die Narkoseflüssigkeit auch im Bereich des Anschlußstückes unterhalb der Grenztemperatur befinden muß.

In einer vorteilhaften Ausgestaltung ist das temperaturempfindliche Schaltglied ein mit einem Fluid gefüllter elastischer Balg, der in thermischem Kontakt mit der Narkoseflüssigkeit steht. Das Fluid kann ein Gas oder eine Flüssigkeit mit hohem Ausdehnungskoeffizienten oder auch die Narkoseflüssigkeit selbst sein, die als Gas- und Flüssigphase innerhalb des Balges vorliegt. Bei Erreichen des Siedepunktes steigt der Dampfdruck, und der Balg dehnt sich aus. Der Balghub ist so zu bemessen, daß nach Unterschreiten der Grenztemperatur das Füllventil und das Belüftungsventil einen für die Flüssigkeits- bzw. Gasströmung ausreichenden Öffnungsquerschnitt haben. Alternativ zu einem elastischen Balg kann auch ein Bimetallelement verwendet werden, dessen Ausdehnungsverhalten auf die Grenztemperatur abgestimmt ist. In einer besonders kostengünstigen Ausführungsform ist das Verschlußteil des Füllventils oder Belüftungsventils direkt mit dem Bimetallelement verbunden.

Eine zweckmäßige konstruktive Ausführung sieht vor, das Füllventil als eine hubbewegliche, im Absperrgehäuse geführte Hülse auszubilden, mit der die Bohrung des Füllkanals verschlossen wird. Das Belüftungsventil besteht aus einem Ventilsitz und einem hubbeweglichen Verschlußteil mit eingelassener Dichtplatte, welche den Belüftungskanal absperrt. Die Hülse und das Verschlußteil sind zur gemeinsamen Betätigung mit einer Stange verbunden, die im Ausgleichsgehäuse geführt ist. Das Verschlußteil wird direkt von dem Balg betätigt.

Die Erfindung wird nachfolgend anhand der Zeichnung erläutert.

Es zeigen:

Fig. 1     einen Längsschnitt durch eine Füllvorrichtung,

Fig. 2     eine Absperrvorrichtung
           - Einzelheit "A" aus Fig. 1 -
           in Schließstellung,

Fig. 3     eine Absperrvorrichtung
           - Einzelheit "A" aus Fig. 1 -
           in Öffnungsstellung.

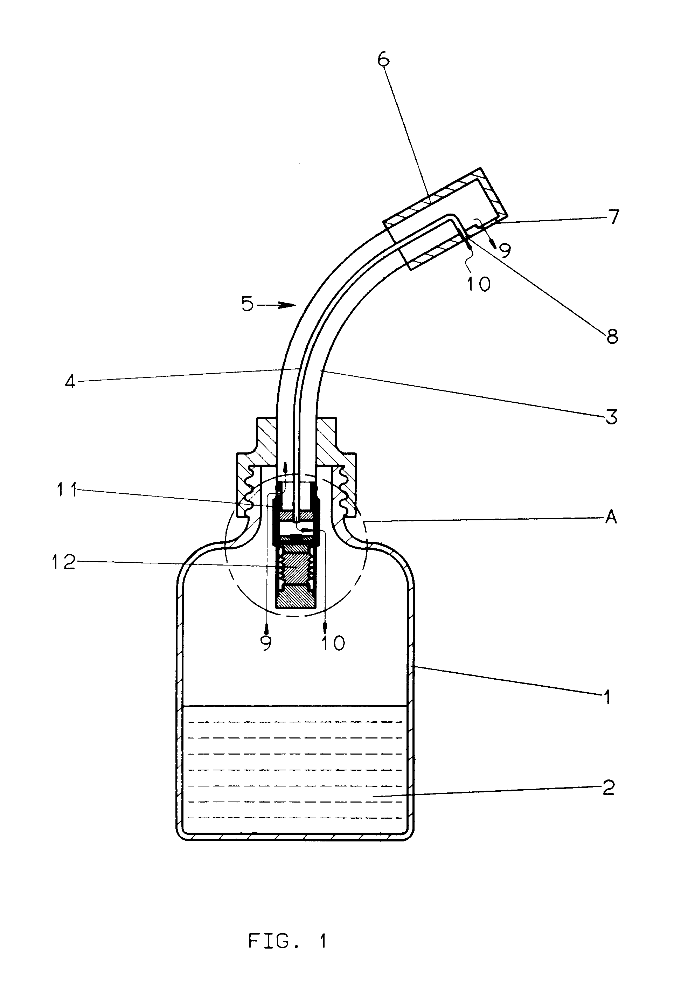

In Fig. 1 ist eine Füllvorrichtung gezeigt zum Befüllen und Entleeren eines nicht dargestellten Narkosemittelverdunsters mit einem Vorratsbehälter (1) für die Narkoseflüssigkeit (2), einem Füllkanal (3), einem Belüftungskanal (4), welche als röhrenförmiges Leitungsstück (5) ausgebildet sind und in einem Anschlußstück (6) münden, das in das entsprechende Gegenstück im Narkosemittel-Verdunster geschoben wird. Das Anschlußstück (6) hat eine Befüllbohrung (7), über die die Narkoseflüssigkeit (2) in den Tank des Narkosemittelbehälters fließt und eine Belüftungsbohrung (8), über die die Belüftung des Vorratsbehälters (1) erfolgt. Der Pfeil (9) gibt den Strömungsweg für die Narkoseflüssigkeit (2), der Pfeil (10) den Gasweg für die Belüftung an. Zum Befüllen wird der Vorratsbehälter (1) umgestülpt, so daß sich die Narkoseflüssigkeit am Hals des Vorratsbehälters (1) befindet und längs des Pfeils (9) in den Narkosemittelverdunster strömt. Ein nicht dargestelltes Umschaltventil am Narkosemittelverdunster verhindert, daß zu Beginn des Füllvorganges Narkoseflüssigkeit (2) in den Belüftungskanal (4) gelangt, indem die Belüftungsbohrung verschlossen wird. Nachdem der Füllkanal (3) mit Narkoseflüssigkeit (2) gefüllt ist, wird der Füllvorgang eingeleitet, indem mit dem Umschaltventil die Belüftungsbohrung (8) geöffnet wird und ein kommunizierender Gas- und Flüssigkeitsaustausch zwischen Narkosemittelverdunster und Vorratsbehälter (1) entsteht. Nach dem Befüllen wird der Vorratsbehälter (1) abgesenkt und das Umschaltventil wieder geschlossen.

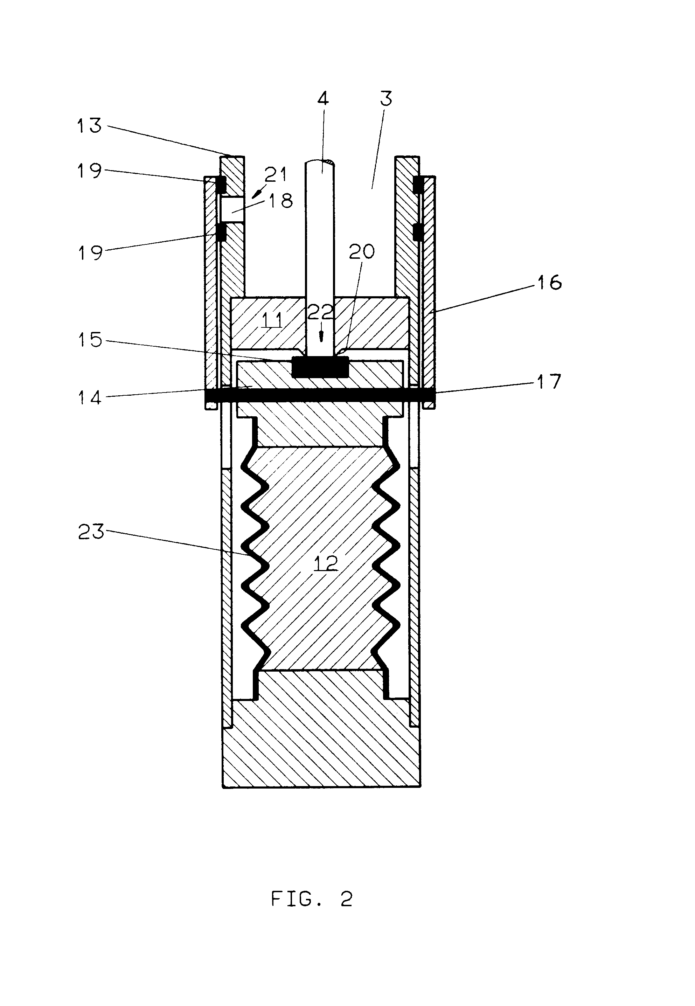

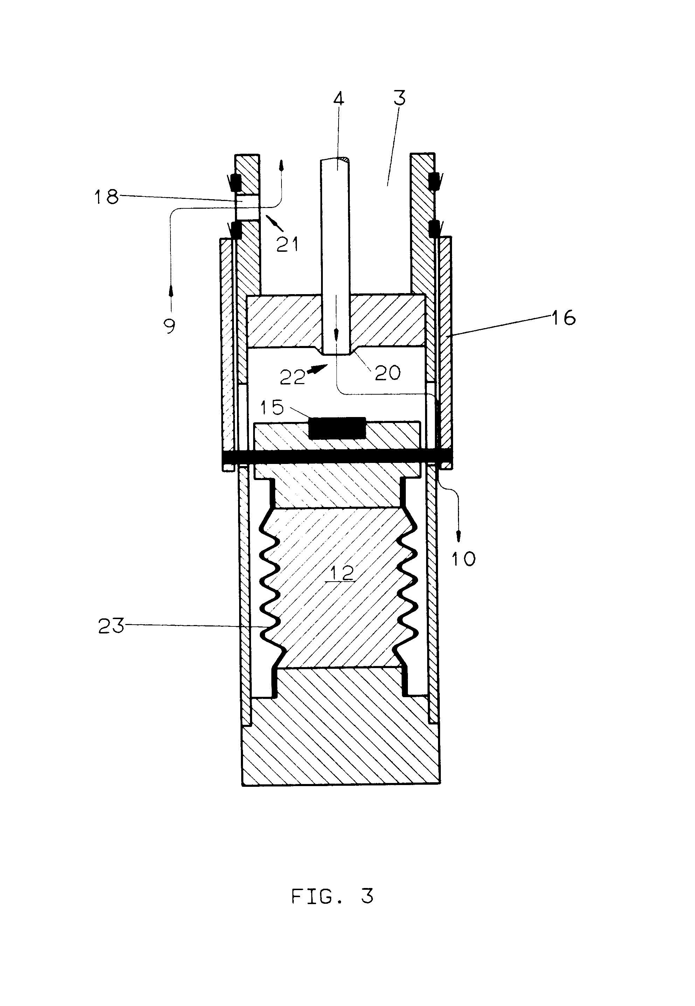

Am Hals des Vorratsbehälters (1) befinden sich eine Absperrvorrichtung (11) und ein temperaturempfindliches Schaltglied (12), die in den Innenraum des Vorratsbehälters (1) ragen und in Fig. 2 und Fig. 3 als Einzelheit "A" veranschaulicht sind.

Fig. 2 zeigt die Absperrvorrichtung (11) in Schließstellung. In ein Absperrgehäuse (13) münden der Füllkanal (3) und der Belüftungskanal (4). Im Absperrgehäuse ist ein elastischer Balg (23) als temperaturempfindliches Schaltglied (12) befestigt, an dessen freiem Ende ein Verschlußteil (14) mit einer Dichtplatte (15) vorgesehen ist. Die Dichtplatte liegt auf einem Ventilsitz (20) auf. Über das Absperrgehäuse (13) ist eine Hülse (16) geschoben, die mit dem Verschlußteil (14) über eine Stange (17) verbunden ist und über eine Bohrung (18) ragt. Zwischen Hülse (16) und Bohrung (18) sind zwei Rundschnurringe (19) als Dichtung vorgesehen. Das Füllventil (21) für den Füllkanal (3) wird gebildet aus der Hülse (16) und der Bohrung (18) im Absperrgehäuse (13) und das Belüftungsventil (22) aus dem Ventilsitz (20) und der Dichtplatte (15). In der gezeigten Stellung sind sowohl das Füllventil (21) als auch das Belüftungsventil (22) verschlossen. Das Füllventil (21) und das Belüftungsventil (22) bilden zusammen die Absperrvorrichtung (11). Beim Absenken der Umgebungstemperatur unterhalb einer Grenztemperatur zieht sich der Balg (23) des Schaltgliedes (12) zusammen, wodurch die Hülse (16) die Bohrung (18) freigibt und die Dichtplatte (15) sich vom Ventilsitz (20) abhebt. Diese Öffungsstellung von Füllventil (21) und Belüftungsventil (22) ist in Fig. 3 veranschaulicht. Längs des Pfeiles (9) kann nun Narkoseflüssigkeit (2) in den Narkosemittelverdunster strömen und umgekehrt zur Belüftung Gas längs des Pfeiles (10) in den Vorratsbehälter gelangen. Der Balg (23) des Schaltgliedes (12) ist aus dünnem Messingblech ausgeführt und zur Hälfte mit der Narkoseflüssigkeit (2) gefüllt. Die Verformung des Balges (23) erfolgt über den sich im Innenraum aufbauenden Dampfdruck.

**Patentansprüche**

1.  Füllvorrichtung zum Befüllen und Entleeren eines Narkosemittelverdunsters mit einer Narkoseflüssigkeit (2) aus einem Vorratsbehälter (1) über einen Füllkanal (3) und einen Belüftungskanal (4), mit einem Anschlußstück (6) zum Narkosemittelverdunster und mit einer Absperrvorrichtung (11), die im Leitungszug von Füllkanal (3) und Belüftungskanal (4) angeordnet ist, dadurch gekennzeichnet, daß die Absperrvorrichtung (11) ein temperaturempfindliches Schaltglied (12) besitzt, durch welches zumindestens der Füllkanal (3) unterhalb einer vorgewählten Grenztemperatur in Öffnungsstellung und oberhalb der vorgewählten Grenztemperatur, jedoch unterhalb des Siedepunktes der Narkoseflüssigkeit (2), in Schließstellung bringbar ist.

2.  Füllvorrichtung nach Anspruch 1, dadurch gekennzeichnet, daß die Absperrvorrichtung (11) ein Füllventil (21) im Füllkanal (3) und ein Belüftungsventil (22) im Belüftungskanal (4) enthält, welche gemeinsam durch das temperaturempfindliche Schaltglied (12) betätigbar sind.

3.  Füllvorrichtung nach Anspruch 1 oder 2, dadurch gekennzeichnet, daß die Absperrvorrichtung (11) innerhalb des Vorratsbehälters (1) angeordnet ist.

4.  Füllvorrichtung nach Anspruch 1 oder 2, dadurch gekezeichnet, daß die Absperrvorrichtung (11) innerhalb des Anschlußstückes (6) angeordnet ist.

5.  Füllvorrichtung nach einem der Ansprüche 1 bis 4, dadurch gekennzeichnet, daß das temperaturempfindliche Schaltglied (12) ein mit einem Fluid gefüllter elastischer Balg (23) ist, der in thermischem Kontakt mit der Narkoseflüssigkeit (2) steht.

6.  Füllvorrichtung nach einem der Ansprüche 1 bis 5, dadurch gekennzeichnet, daß das Füllventil (21) eine hubbewegliche, im Absperrgehäuse (13) geführte Hülse (16) aufweist, mit der die Bohrung (18) des Füllkanals (3) verschließbar ist, und daß das Belüftungsventil (22) aus dem Ventilsitz (20) und dem hubbeweglichen Verschlußteil (14) besteht, mit welchem der Belüftungskanal (4) verschließbar ist, wobei die Hülse (16) und das Verschlußteil (14) über eine Stange (17) gemeinsam betätigt sind und das Verschlußteil (14) in Wirkverbindung mit dem Balg (23) steht.

FIG. 1

FIG. 2

FIG. 3

## EINSCHLÄGIGE DOKUMENTE

EP 91109635.2

| Kategorie | Kennzeichnung des Dokuments mit Angabe, soweit erforderlich, der maßgeblichen Teile | Betrifft Anspruch | KLASSIFIKATION DER ANMELDUNG (Int. Cl⁵) |
|---|---|---|---|
| A | EP - A2 - 0 242 979 (BOC GROUP) * Gesamt; insbesondere Spalte 2, Zeile 30 - Spalte 4, Zeile 11 * -- | 1 | A 61 M 16/18 |
| D,A | DE - A1 - 3 720 326 (DRÄGERWERK) * Gesamt * -- | 1 | |
| D,A | DE - B2 - 1 657 174 (FRASER SWEATMAN INC.) * Gesamt * -- | 1 | |
| A | DE - B - 1 922 404 (DRÄGERWERK) * Fig. 1,2; Spalte 5, Zeilen 27-33; Spalte 6, Zeilen 30-36 * ---- | 1 | |

**RECHERCHIERTE SACHGEBIETE (Int. Cl⁵)**

A 61 M

Der vorliegende Recherchenbericht wurde für alle Patentansprüche erstellt.

| Recherchenort | Abschlußdatum der Recherche | Prüfer |
|---|---|---|
| WIEN | 24-10-1991 | LUDWIG |